# EUROPÄISCHE PATENTSCHRIFT

(11) **EP 0 971 636 B1**
(45) Veröffentlichungstag und Bekanntmachung des Hinweises auf die Patenterteilung: **26.10.2005**
(21) Anmeldenummer: 98928114.2
(22) Anmeldetag: 01.04.1998
(51) Int. Cl.: A61B 18/00

(54) **VORRICHTUNG ZUR ERFASSUNG DES KATHETER-GEWEBEKONTAKTES SOWIE VON WECHSELWIRKUNGEN MIT DEM GEWEBE BEI DER KATHETERABLATION**
DEVICE FOR DETECTING CATHETER-TISSUE CONTACT AND INTERACTION WITH TISSUE DURING CATHETER ABLATION
DISPOSITIF DE DETECTION DU CONTACT DU TISSU AVEC UN CATHETER, AINSI QUE DES INTERACTIONS AVEC LE TISSU LORS DE L'ABLATION PAR CATHETER

(30) Priorität: 01.04.1997 DE 19713234; 17.09.1997 DE 19740976
(43) Veröffentlichungstag der Anmeldung: 19.01.2000
(73) Patentinhaber: Muntermann, Axel, 35578 Wetzlar (DE)
(72) Erfinder: Muntermann, Axel, 35578 Wetzlar (DE)
(74) Vertreter: Blumbach - Zinngrebe
(86) Internationale Anmeldenummer: PCT/DE1998/000932
(87) Internationale Veröffentlichungsnummer: WO 1998/043547

(56) Entgegenhaltungen:
- EP-A- 0 391 233
- WO-A-96/41569

## Beschreibung

Die Erfindung betrifft eine Vorrichtung zur Erfassung des Kontaktes eines in einem Gefäß eines Patienten, insbesondere in der Blutbahn eines Patienten, angeordneten Katheters zu dem Gewebe und ferner eine Vorrichtung zur Erfassung der Wechselwirkung von Hochfrequenzenergie mit dem Gewebe des Patienten.

Bei vielen medizinischen Anwendungen oder Behandlungen ist es für den Arzt von höchster Wichtigkeit, den Kontakt eines Instrumentes zu dem Körper des Patienten mit hoher Exaktheit zu erfassen, zu verfolgen und in manchen Fällen auch aufzuzeichnen, da hiervon häufig der Behandlungserfolg in wesentlichem Maße abhängt.

Insbesondere bei der Katheterablation ist die erwünschte Behandlungswirkung in der Regel nur dann zu erzielen, wenn ein Kontakt zwischen dem Ablationkatheter und dem zu behandelnden Gewebe des Patienten während der gesamten Einwirkungsdauer der abgegebenen.Ablationsleistung sichergestellt werden kann.

Bisher wurden zur Erfassung des Katheter-Gewebekontaktes Widerstandsmessungen zwischen zumindest zwei Katheterelektroden oder den Katheterelektroden und einer amKörper des Patienten angeordneten indifferenten Elektrode vorgenommen. Diese Vorgehensweise ist jedoch, insbesondere bei der Hochfrequenz-Katheterablation, nachteilig, denn die Widerstandsmessung ist prinzipiell mit einem Stromfluß durch den zu messenden Bereich verbunden. Hierdurch werden aber zusätzliche Ströme bei der Ablation eingeführt, die, beispielsweise bei der Erfassung der EKG-Signale, äußerst störend sein können. Verwendet man Gleichstromwiderstandsmeßverfahren, um hochfrequente, die EKG-Erfassung beeinflussende Signale zu vermeiden, werden unerwünschte elektrolytische Wirkungen erzeugt, die zu einer weiteren chemischen Belastung des Patienten führen. Darüber hinaus sind Widerstandsmessungen überall da schwierig durchzufahren und störanfällig, wo korrosive. Einflüsse und Oberflächenverschmutzungen den Stromfluß behindern bzw. das Meßergebnis verfälschen. Eine derartige Situation liegt jedoch gerade im Bereich der medizinischen Anwendungen vor, da hier das Blut oder Körperflüssigkeiten des Patienten Salze bzw. koagulierende Substanzen aufweisen, die mit der Oberfläche von Elektroden oder Kontakten auf unerwünschte Weise wechselwirken können. Außerdem ist durch die Impedanzmessung während der Ablation der Gewebekontakt nur schwer zu beurteilen, da viele Faktoren den Impedanzwert beeinflussen können.

So ist z.B. in dem Dokument WO 97/21387 (PCT/DE96/00638) eine Vorrichtung beschrieben, bei welcher mittels einer Messung der Impedanz der Katheterelektroden gegenüber einer am Patienten angeordneten indifferenten Elektrode Aufschluß über die richtige Lage des aktiven Katheterabschnitts relativ zu dem zu behandelnden Gewebe gewonnen wird. Hierzu wird die Impedanz der Elektroden in Bezug auf eine weitere Elektrode als Maß für den Gewebekontakt erfasst, angezeigt und/oder zeitlich zugeordnet gespeichert.

Ferner sind aus dem Dokument WO 96/41569 eine Vorrichtung und ein Verfahren zur thermischen Ablation bekannt, bei welchen eine Sondenspitze und eine Platte mit unterschiedlicher Austrittsarbeit verwendet werden, um eine galvanische Zelle zu bilden. Die galvanische Zelle wird mittels einer Nebenschlussimpedanz stark belastet, um einen Stromfluss zu erzeugen, mittels welchem ein Signal bereit gestellt wird, das die Gewebetemperatur an der Ablationsstelle reflektiert.

Der Erfindung liegt folglich die Aufgabe zugrunde, die vorstehend erwähnten Nachteile zu vermeiden und zu einer verbesserten Erfassung des Kontaktes zwischen Katheter und Gewebe des Patienten beizutragen.

Diese Aufgabe wird auf höchst überraschende Weise bereits durch eine Vorrichtung nach Anspruch 1 gelöst.

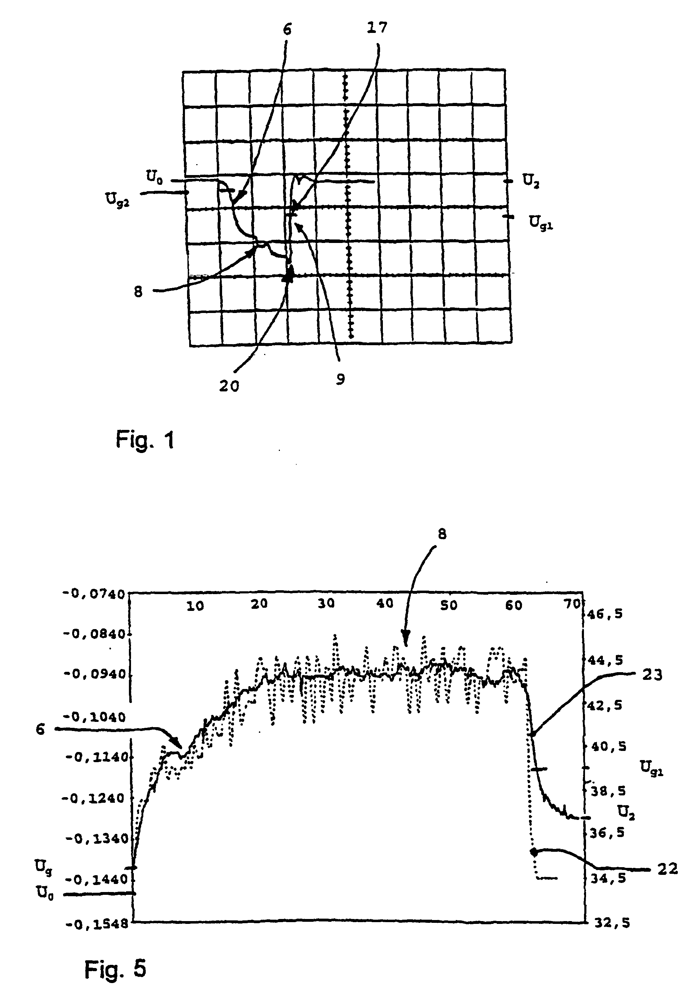

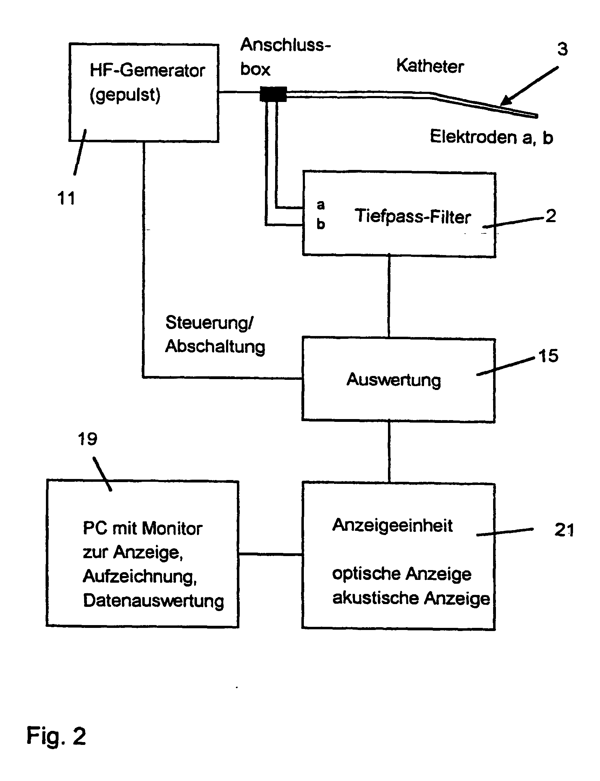

Der Erfinder hat in überraschender Weise festgestellt, daß in vielen Fällen beim Anlegen von Elektroden an das Gewebe von Patienten, insbesondere bei blutumspülten Gewebe, Spannungen zwischen den Elektroden und insbesondere während der HF-Ablation auftreten. Die vorzugsweise metallische Elektrode eines Ablationskatheters erzeugt beispielsweise das in Fig. 1 dargestellte Spannungssignal, welches gemäß der Erfindung in dem in Fig. 2 dargestellten Meßaufbau erhalten wurde.

Auf höchst überraschende Weise hat der Erfinder festgestellt, daß ein in der Blutbahn angeordnetes Katheter ein hier mit U₀ bezeichnetes, zunächst nur sehr schwaches Spannungssignal erzeugt, wohingegen ein abrupter Spannungsanstieg dann erfolgt, wenn die Katheterelektrode mit dem Gewebe in Kontakt tritt. Das hierbei gewonnene Spannungssignal stellt mit dessen Momentanwert bzw. Amplitude ein Maß für die Güte des Katheter-Gewebekontaktes dar und kann ohne Verwendung zusätzlicher äußerer Ströme erfaßt werden. Folglich werden in erfindungsgemäßer Weise keine elektrolytischen Vorgänge auftreten, und es ist davon auszugehen, daß weitere Messungen, wie beispielsweise die Aufzeichnung von EKG-Signalen, nicht negativ beeinflußt werden.

Darüber hinaus hat es sich gezeigt; daß die erfindungsgemäße Spannungsmessung der herkömmlichen Widerstandsmessung auch in Bezug auf die gewonnenen Meßdaten deutlich überlegen ist. Zum einen ist die Spannung ein äußerst genaues Maß für den Kontakt der Katheterelektrode zum Gewebe, während eine Impedanzmessung während der Ablation nur bedingt auf den Gewebekontakt des Katheters schließen läßt, und zum anderen tritt dieses Signal nahezu ohne jegliche zeitliche Verzögerung auf, welches dieses zur Echtzeitmessung geeignet macht.

Auf besonders überraschende Weise hat sich ferner herausgestellt, däß die Höhe des gemessenen Signals sehr exakt mit der Temperatur des Gewebes, insbesondere des während einer Abladierung sich erwärmenden Gewebes, korreliert. Mit parallelen Meßverfahren konnte gezeigt werden, daß mit der Erfindung Temperaturwerte des am Katheter anliegenden Gewebes mit einer Genauigkeit von +./- 1 °C meßbar waren. Ferner waren bereits mit einfachen Mitteln, d.h. einfachen hochohmigen Spannungsmeßeinrichtungen, Genauigkeiten von +/- 2 °C meßbar. Hierbei stand die Temperatur regelmäßig linear mit der gemessenen Spannung im Verhältnis, welches es ermöglichte, standardisierte Spannungsmeßwerte demselben Ablationskatheter oder einer Gruppe baugleicher Katheter zuzuordnen.

Da Spannungen prinzipiell auch stromfrei meßbar sind, beispielsweise durch Aufprägen einer gleichhohen Gegenspannung oder durch Messung mit sehr hochohmigen Meßverstärkern, spielen Übergangswiderstände von Kontakten innerhalb der Meßstrecke eine weit weniger wichtige Rolle als bei allen herkömmlichen Meßverfahren. Folglich ist die erfindungsgemäße Vorrichtung bzw. das erfindungsgemäße Verfahren gegenüber den herkömmlichen Verfahren weniger Störungen ausgesetzt und zuverlässiger sowie sicherer anwendbar. Darüberhinaus scheinen thermische (Durchgangs-) Widerstände sowie thermische Kapazitäten des Katheters selbst weit geringere Einflüsse auf die Genauigkeit sowie Geschwindigkeit der Messung der Temperatur zu haben.

Auf weiter höchst überraschende Weise hat sich bei der Katheterablation selbst herausgestellt, daß deren Ergebnisse erheblich verbessert werden konnten. Wird beispielsweise wie bei der herkömmlichen Kathetertemperatursteuerung die Kathetertemperatur als Regelgröße und somit als Maß für die Energieabgabe verwendet, kann es vorkommen, daß bei relativ kaltem Katheter, beispielsweise bei einer Temperatur von nur etwa 40 °C, bereits dann eine deutliche Läsion im Gewebe hervorgerufen wird, wenn der Katheter in sehr engem Kontakt mit dem Gewebe stand und hierdurch einen großen Teil der Ablationsenergie in dieses leiten konnte.

Die herkömmliche, reine Temperatursteuerung mit thermischen Sensoren hätte in diesem Fall die Läsion nicht erfaßt. Darüber hinaus kann es selbst bei hoher Kathetertemperatur dann zu nur geringer Läsions- bzw. Ablationswirkung kommen, wenn der Katheter-Gewebekontakt sehr schlecht ist. In beiden Fällen konnte es bei den herkömmlichen Verfahren und Vorrichtungen zu einer ungenauen bzw. falschen Aussage über den Behandlungserfolg kommen.

Bei der Erfindung wird jedoch davon ausgegangen, daß durch die auftretenden und erfaßten Spannungssignale stets sichergestellt wird, daß die Energieabgabe im wesentlichen in das Gewebe erfolgt, und folglich kann bei der Erfindung die vom Katheter abgegebene Leistung direkt und exakter einer Behandlungswirkung zugeordnet werden. Ferner kann, nachdem der abrupte Spannungsanstieg den Katheter/Gewebekontakt erfaßt hat, die absolute Höhe des Spannungssignals zur sehr genauen Temperaturmessung benutzt werden.

Weiterhin ist es möglich, das auftretende Spannungssignal zur Steuerung bzw. Überwachung des Ablationsvorgangs selbst zu verwenden. Werden beispielsweise bei der Hochfrequenz-Ablation die Spannungssingale während der Abgabe der Hochfrequenzleistung erfaßt, und wird bei Unterschreiten bzw. Überschreiten die Abschaltung oder zumindest die Verminderung der Abgabe der Hochfrequenzleistung bewirkt, wird stets gewährleistet, daß die Hochfrequenzleistung im wesentlichen in das Patientengewebe abgegeben wurde, und es kommt zu einer Behandlung mit insgesamt kühleren Katheterelektroden und erhöhter Wirkung.

Es ist darüberhinaus vorteilhaft, das Spannungssignal zu integrieren und somit zu einer Aussage über die Wechselwirkung, d. h. über die einwirkende Temperatur und die Zeit der Behandlung, zu gelangen.

Darüber hinaus treten Verschmutzungen des Katheters durch Koagulation wegen der nunmehr aufgrund der exakteren Messung möglich gewordenen niedrigeren Kathetertemperaturen nur in sehr viel geringerem Maße auf, und es wird ein durch derartige Verschmutzungen verursachter Behandlungsabbruch mit der damit einhergehenden erhöhten Patientenbelastung vermieden.

Wird die Abschaltung oder zumindest die Verminderung der Abgabe der Hochfrequenzleistung bei einem raschen Anstieg oder Abfall der Potentialwerte bewirkt, kann hierdurch auch die Vaporisierung des zu behandelnden Gewebes verhindert oder zumindest stark beschränkt werden, da nunmehr eine sehr schnelle Regelung möglich ist und es nicht mehr zur ungewollten Überhitzung des Gewebes kommt.

In höchst überraschender Weise hat sich ferner herausgestellt, daß die erfindungsgemäßen Potentiale bzw. Spannungssignale während der Durchführung der Hochfrequenz-Ablation, unabhängig davon, ob die Hochfrequenzsignale zur Ablation entweder kontinuierlich oder gepulst angelegt werden, stets sehr sicher erfaßt werden können. Wird die von dem Katheter abgegebene Hochfrequenzleistung zeitlich integriert, solange die Spannungssignale oberhalb eines vorgebbaren Grenzwertes liegen, welcher sicherstellt, daß ein Katheter/Gewebekontakt vorhanden ist, und wird die integrierte Leistung als bis zu diesem Zeitpunkt in das Gewebe abgegebene Ablationsenergie berechnet, kann dem behandelnden Arzt nicht nur das Fortschreiten des Behandlungserfolges angezeigt werden, sondern es kann mit einer entsprechend programmierten Steuereinrichtung die Behandlung bei Erreichen eines vorgegebenen Energiewertes automatisiert durch Abschaltung oder Verminderung der Hochfrequenzleistung beendet werden. Hierdurch kann eine weit höhere Behandlungssicherheit bereitgestellt werden, als dies bei den bisherigen Verfahren möglich war. Erstmalig ist es hierdurch möglich, bereits im Verlauf der Behandlung die Ablationswirkung durch Integration der abgegebenen Leistung zu erfassen und dem behandelnden Arzt in Echtzeit anzuzeigen.

Darüberhinaus kann der behandelnde Arzt in weiterer erfindungsgemäßer Ausgestaltung Gewebetiefen vorgeben, die dem zeitlichen Integral der gemessenen Potentiale zugeordnet werden. Es kann dann die erfindungsgemäße Vorrichtung entweder den gesamten Ablationsvorgang nach Erreichen des vorgegebenen Wertes beenden, oder es können bei Kathetern mit mehreren Elektroden lokal zugeordnet Abschnitte des Katheters, innerhalb derer die vorgegebenen Werte erreicht wurden, abgeschaltet oder diesen geringere Leistung zugeführt werden.

Werden hierzu in bevorzugter erfindungsgemäßer Weise Katheter mit mehreren Ablationselektroden verwendet, können die Signaldaten der jeweiligen speziellen Elektrode zugeordnet, erfaßt, berechnet sowie angezeigt werden, und es kann der Behandlungsablauf lokal dem Behandlungsort zugeordnet programmierbar beendet werden. Hierdurch können bereits vor Behandlungsbeginn für die Behandlung optimierte Daten auch lokal zugeordnet vorgegeben werden, und es kann eine in Bezug auf den jeweiligen Patienten optimierte Behandlung durchgeführt werden.

Die Erfindung wird nachfolgend anhand bevorzugter Ausführungsformen und unter Bezugnahme auf die beigefügten Zeichnungen detaillierter beschrieben.
Es zeigen:
- Fig. 1: die Signalform der erfindungsgemäßen Spannung, wie sie bei einem zeitlich begrenzten Katheterelektroden-Gewebekontakt in einem System mit indifferenter Platinelektrode sowie Platin-Katheterelektrode auftritt,
- Fig. 2: eine schematische Darstellung zur Durchführung der Erfindung mit einem Katheter sowie einer Vorrichtung, die beispielsweise der in der PCT/DE96/00638 beschriebenen entspricht, jedoch in erfindungsgemäßer Weise weiter ausgestaltet wurden,
- Fig. 3: einen Labor Meßaufbau zur Standardisierung bzw. Eichung sowie Meßdatengewinnung vermittels eines Schweineherzens,
- Fig. 4: ein Tiefpaßfilter, welches bereits zusammen mit einem Oszilloskop zur Gewinnung der erfindungsgemäßen Spannungssignale verwendbar ist,
- Fig. 5: einen Vergleich der erfindungsgemäß gewonnenen Potentialwerte mit lokal zugeordneten, an einem Ablationskatheter gewonnenen Temperaturwerten während der Katheterablation in einem lebenden Herzen,
- Fig. 6: einen Vergleich der Werte des zeitlichen Integrals der erfindungsgemäß gewonnenen Potentialwerte mit bei der Katheterablation erreichten Läsionstiefen bei verschiedenen Temperaturen des Ablationskatheters.

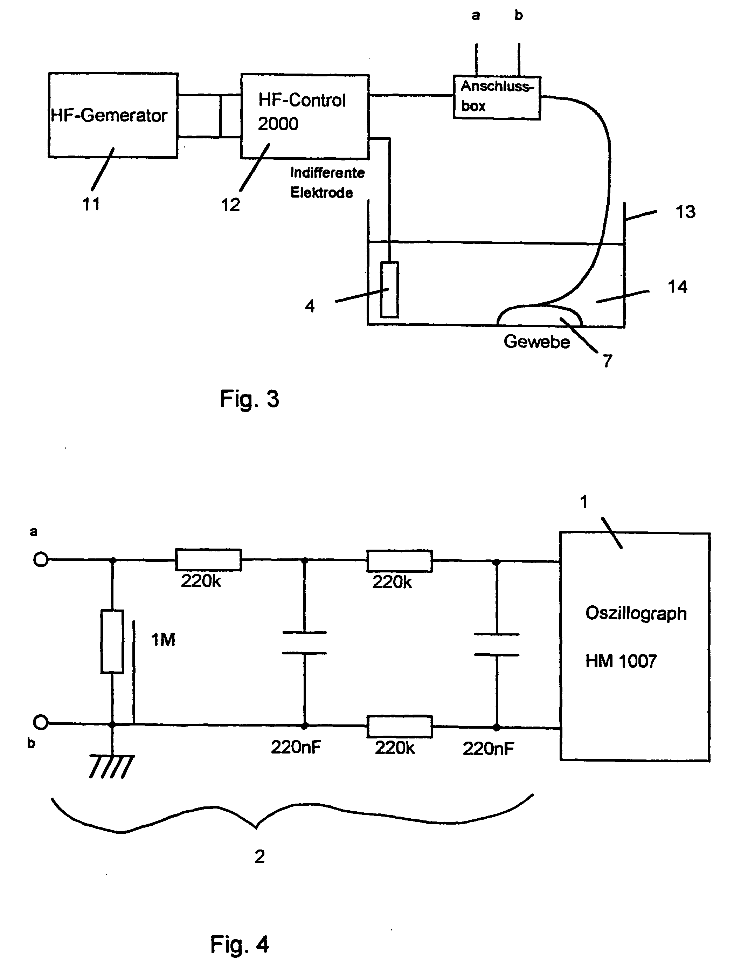

Die Erfindung wird nachfolgend unter Bezugnahme auf bevorzugte Ausführungsformen und zunächst unter Bezugnahme auf die Fig. 1 und 4 dargestellt.

In einer ersten, zunächst zur Erläuterung äußerst einfach gehaltenen erfindungsgemäßen Ausführungsform wird das in Fig. 1 dargestellte Spannungssignal mit der Spannungsmeßvorrichtung 1, der ein Tiefpaßfilter 2 vorgeschaltet wurde, gewonnen.

Die Spannungsabgriffe des Tiefpaßfilters a und b wurden mit Platinelektroden eines bipolaren Katheters verbunden, wie es beispielsweise in der PCT/DE96/00638 beschrieben wurde. Das Katheter 3 braucht- jedoch für die Durchführung der vorliegenden Erfindung generell keine zusätzlichen Mittel zur Erfassung der Kathetertemperatur, wie beispielsweise thermische Sensoren, aufzuweisen, obwohl dies durch die Erfindung nicht ausgeschlossen wird.

Alternativ zum Abgriff der Spannung an den Katheterelektroden a und b kann die Spannung auch zwischen der oder den indifferenten Elektroden 4 und einer der Elektroden a, b des Katheters 3 erfaßt werden, oder dem Ort der jeweiligen Katheterelektrode a, b zugeordnet erfaßt werden.

Das Tiefpaßfilter 2 der ersten erfindungsgemäßen Ausführungsform ist mit einem Speicheroszilloskop des Typs Hameg HM 1007 verbunden, welches in gewohnter Weise eine zweidimensionale Anzeigevorrichtung.zur zeitlichen Darstellung von Spannungsverläufen darstellt.

Hiermit wurde zunächst mit dem in Fig. 3 dargestellten Meßaufbau die in Fig. 1 gezeigte Signalform des zeitlichen Spannungsverlaufs erhalten, die Zeitbasis betrug 20s TIME/DIV und die angezeigte Spannung entsprach 10mV VOLTS/DIV. Der zunächst niedrige,ebene Abschnitt 5 des Grundsignals Uo stellt die Spannung bei im Blutstrom befindlichem Katheter 3 dar und zeigt eine deutlich ausgeprägte Signalflanke 6, sobald das Katheter 3 mit Gewebe 7 in Kontakt tritt.

Die Signalschulter 8, welche sich nach dem abrupten Anstieg der Signalflanke 6 im wesentlichen eben erstreckt, zeigt eine zeitliche Modulation, die auf den mechanischen Katheter-Gewebekontakt zwischen der Katheterelektrode b und dem Gewebe 7 in Bezug auf die indifferente Elektrode 4 oder die Elektrode a zurückzuführen sein kann oder mit der Erzeugung und dem Transport chemischer Substanzen, welche elektrische Potentiale am Ort des Katheters hervorrufen, zusammenhängen.

Wird der Katheter vom Gewebe entfernt, kommt es zur abfallenden Signalflanke 9, die in ein gegenüber dem Grundsignal 0 leicht erhöhtes Grundsignal U₂ abklingend übergeht.

Der abrupte Anstieg 6 sowie Abfall 9 der Signalflanke zeigt jedoch das Entstehen sowie Beenden eines Katheter/Gewebekontaktes mit hoher Sicherheit an.

Der zur Gewinnung der in Fig. 1 dargestellten Signalform verwendete Aufbau ist in Fig. 3 schematisch in Form eines stationären Meßaufbaus, der zur Standardisierung und Eichung verwendbar ist, gezeigt. Ein HF-Generator 11 ist mit dem in der vorstehend zitierten PCT-Anmeldung beschriebenen Gerät zur kontrollierten Abgabe von HF-Leistung an den Katheter 3, welcher mehrere Ablationselektroden aufweist, verbunden und führt den Elektroden des Katheters 3 gepulste Hochfrequenzleistung zu.

In alternativen erfindungsgemäßer Ausgestaltung kann jedoch die Hochfrequenzleistung dem Katheter 3 auch zeitlich kontinuierlich zugeführt werden.

Innerhalb eines Trogs 13 sind die indifferente Elektrode 4 sowie Gewebe 7 angeordnet, an welchem mittels des Katheters 3 Ablationsvorgänge vorgenommen werden. Der Trog 13 kann entweder mit Blut oder mit einer geeigneten anderen Flüssigkeit gefüllt sein, um die im Körper des Patienten herrschenden Bedingungen zu simulieren.

Die indifferente Elektrode 4 sowie die Elektroden a und b des Katheters 3 sind platinbeschichtet oder bestehen aus Platin und werden unmittelbar vor dessen verwendung mit Formalin oder Formaldehydgas derart gereinigt, daß im wesentlichen keinerlei Oberflächenrückstände am Katheter bzw. der indifferenten Elektrode 4 verbleiben.

Es hat sich herausgestellt, daß die Reinigung mit Formalin bzw. Formaldehydgas zu sehr guten und genau reproduzierbaren Meßergebnissen führte, welche über einen Zeitraum von mehreren Tagen sehr exakt nachvollziehbar waren. Jedoch liegt es im Rahmen der Erfindung, auch andere Reinigungsverfahren zu verwenden, um zu einer im wesentlichen rückstandsfreien Elektrodenoberfläche zu gelangen. Bei sterilisierten Kathetern können auch über sehr viel längere Zeiträume sehr gute Meßergebnisse erhalten werden.

Ferner wird auch die Verwendung anderer Metalle bzw. chemischer Substanzen, die in der Lage sind; die erfindungsgemäße Spannung oder ähnliche, beim Inkontakttreten und/oder Inkontaktstehen der Katheterelektroden mit dem Gewebe auftretender Spannungen zu erzeugen, nicht ausgeschlossen. Hierzu wird in erfindungsgemäßen Sinne jedoch ohne Beschränkung der Allgemeinheit davon ausgegangen, daß während der Katheterablation freie Radikale oder zumindest chemische Substanzen erzeugt werden, welche die erfindungsgemäßen Potentiale hervorrufen. Wird ferner davon ausgegangen, daß bei starker Wechselwirkung der Hochfrequenzleistung mit dem Gewebe ein vermehrter Austritt der vorstehend erwähnten Substanzen aus dem Gewebe stattfindet, kann die erfindungsgemäße Vorrichtung direkt Aussagen über die im Gewebe erzeugte Schädigung bzw. Läsionswirkung bereitstellen.

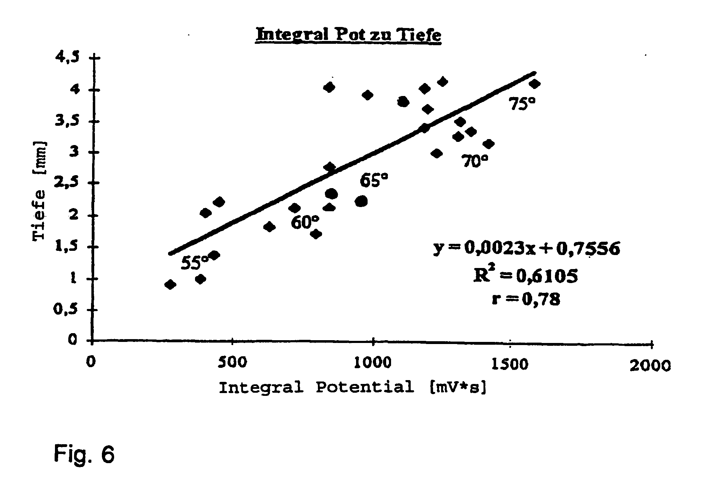

Nachfolgend wird auf Fig. 5 Bezug genommen, welche den Vergleich der erfindungsgemäßen Potentialwerte mit Temperaturen, die zeitgleich am Ort der Potentialmessung erfaßt wurden; darstellt. Die Übereinstimmung des gestrichelt dargestellten Temperaturwertes mit dem durchgezogen dargestellten Potentialwert 23 stimmt erwartungsgemäß mit dem vorstehend angegebenen Modell gut überein, da auch chemische Potentiale weitgehend temperaturabhängig sind. Fällt jedoch die Temperatur nach der Behandlung wieder ab, wie auf der rechten Seite von Fig. 5 dargestellt, so sinkt das Potenial nur auf den erhöhten Wert U₂ ab, der durch das Vorhandensein der vorstehend diskutierten chemischen Substanzen gut erklärbar ist.

Eine Unterscheidung zwischen Verlust des mechanischen Kontaktes und Abfall der Temperatur ist leicht dadurch möglich, daß die abgegebene Leistung erfaßt und hieraus ein Schwellenwert gebildet wird. Dieser Schwellenwert gibt den minimal zu erwartenden Spannungswert an und kann bei Unterschreitung die Abschaltung der Abgabe von Ablationsenergie bewirken.

Eine weitere deutliche Stützung des Erklärungsmodells der Potentialentstehung findet sich in Fig. 6, gemäß welcher die Abhängigkeit der Tiefe der im Gewebe eines Patienten bei der HF-Ablation erzeugten Läsionen von dem zeitlichen Integral der auftretenden Poteniale dargestellt ist. Über einen Temperaturbereich des Ablationskatheters von über 20 °C, nämlich von weniger als 55 °C bis zu mehr als 75 °C Ablationstemperatur, sind diese Werte gut miteinander korreliert. Folglich kann bei bekannten Kathetereigenschaften in vielen Fällen bereits das zeitliche Integral des Potentials ausreichen, ohne daß zusätzlich Temperaturwerte erfaßt werden müßten, um eine geeignete Aussage über den Behandlungsverlauf zu erhalten.

Derartige Katheter könnten auf thermische Sensoren verzichten und folglich einfacher, kostengünstiger und mit geringerem Durchmesser hergestellt werden.

Es liegt darüberhinaus im Rahmen der Erfindung, für einfachere Ausführungsformen die abgegebene Leistung des HF-Ablationsgenerators ohne Temperaturmessung derart zu beschränken, daß vorgegebene Maximaltemperaturen niemals überschritten werden, so daß auch in diesem Falle eine Unterdrückung von unerwünschten Koagulationsvorgängen sowie eine Vaporisierung von Gewebe unterbleibt.

Um die Genauigkeit der Messung bzw. die Korrelation der in Fig. 6 dargestellten Größen zu verbessern, kann durch die Auswertungseinheit 15 auch eine mit der abgegebenen Leistung gewichtete oder funktional verknüpfte Messung der Potentialwerte, vorzugsweise in Echtzeit, vorgenommen werden.

Eine weitere erfindungsgemäße Ausführungsform ist in Fig. 2 dargestellt, in welcher der in der vorstehend zitierten PCT-Anmeldung beschriebene, gepulst betriebene Generator 11 mit dem Katheter 3 sowie dessen Katheterelektroden a und b verbunden ist. Die Katheterelektroden a und b sind entweder die Ablationselektroden selbst oder in deren Nähe angeordnete, den jeweiligen Ablationselektroden zugeordnete Meßelektroden, die mit dem Tiefpaßfilter 2 verbunden sind, welches sowohl das Hochfrequenzsignal als auch Brummsignalstörungen ausfiltert.

Die nachgeschaltete Auswertungseinheit 15 umfaßt die Spannungsmeßeinrichtung 1, die wie bei der ersten erfindungsgemäßen Ausführungsform einen hochohmigen Meßeingang von wenigstens mehr als 100 kΩ und vorzugsweise mehreren MΩ Eingangswiderstand aufweist, um jeglichen zusätzlichen Stromfluß zum Ablationsvorgang sowie von weiteren Meßvorgängen auf einen im wesentlichen nicht mehr meßbaren Wert zu unterdrücken. In erfindungsgemäßem Sinne ist eine hochohmige Spannungsmessung eine Spannungsmessung, die bei der Durchführung der Ablation oder der Messung von beispielsweise EKG-Signalen nicht mehr auffällt bzw. nicht mehr nachweisbar ist.

Bei einer besonders bevorzugten Ausführungsform, beispielsweise einem Speicheroszilloskop, das mit einem Personal-Computer als Steuereinrichtung verbunden sein kann, weist der Eingangswiderstand etwa 1 MΩ auf, und dessen Eingangskapazität beträgt nicht mehr als 30 pF.

Die mit der Spannungsmeßvorrichtung 1 gewonnenen Meßdaten werden in der Auswertungseinheit 15 aufgezeichnet und gespeichert. Die Daten können entweder in Echtzeit oder durch Auslesen aus dem Speicher der Auswertungseinheit 15 in zweidimensionaler Form an einer Anzeigeinheit 21 dargestellt und etwa zu Vergleichszwecken oder zur Bewertung des Behandlungserfolgs wiedergegeben werden.

Die Darstellung kann hierbei als momentanes Spannungssignal, als Balkendiagramm oder auf beliebige andere, für den behandelnden Arzt ergonomisch günstige Weise erfolgen.

Ferner kann eine der Auswertungseinheit 15 zugeordnete Steuereinrichtung 16 die Abschaltung oder Verminderung der vom HF-Generator 11 abgegebenen Leistung bewirken, wenn das gemessene Spannungssignal unter einen vorgegebenen. Grenzwert U_{g1} 17 fällt, um derart zu verhindern, daß der Katheter oder dessen Umgebung aufgeheizt wird, ohne hierbei die gewünschte Ablation zu erzeugen. Der Grenzwert U_{g1} 17 kann in Abhängigkeit von der jeweils abgegebenen momentanen Ablationsleistung oder integrierten abgegebenen Ablationsleistung, d.h. abgegebenen Ablationsenergie, verändert und somit sehr genau vorgegeben werden. Hierdurch wird eine Verschmutzung des Katheters durch koagulierte. Substanzen in erheblichem Maße vermindert bzw. vermieden.

Darüber hinaus kann die Steuereinrichtung 16 zu Beginn des Ablationsvorgangs beim Überschreiten eines weiteren Grenzwertes U_{g2} 18 beginnen, die vom HF-Generator 11 abgegebene Leistung so lange zu integrieren, bis das Spannungssignal entweder unter den Grenzwert U_{g1} 17 oder unter einen beliebigen anderen vorgebbaren Wert abfällt. Hierdurch wird die der Energieabgabe in das Gewebe zugeordnete Größe erfaßt, die es dem behandelnden Arzt gestattet, Aussagen über die Ablationswirkung zu treffen. Ferner liegt es im Rahmen der Erfindung, die Integration der vom HF-Generator abgegebenen Leistung 11 mit der Höhe des Spannungssignals multipliziert durchzufahren, um zu einer verbesserten oder korrigierten Aussage über die Ablationswirkung zu gelangen.

Es liegt ebenfalls im Rahmen der Erfindung, weitere Funktionswerte in Abhängigkeit von der momentanen Spannung U₁ bei der Integration der vom HF-Generator abgegebenen Leistung mit zu berücksichtigen, wie beispielsweise die bei Standardmessungen ermittelten tabellarischen Funktionswerte der Läsionswirkung für einen standardisierten Katheter 3 bzw. einen individuellen Katheter 3.

Steht ein gewonnener Parametersatz für einen bestimmten Kathetertyp oder ein individuelles Katheter zur Erfassung der Läsionswirkung zur Verfügung, kann auch die Tiefe der Läsion als Funktion der abgegebenen Energie dargestellt und zur Abschaltung des Läsionsvorgangs verwendet werden. Hierdurch wird dem behandelnden Arzt der jeweiligen Katheterelektrode zugeordnet die Möglichkeit gegeben, örtlich verschiedene Tiefen bzw. bestimmte Tiefen und Längen der erzeugten Läsionen zu definieren, an der Steuereinrichtung 16 einzustellen und nachfolgend während der Behandlung automatisiert umzusetzen.

Anstelle der automatisierten Abschaltung kann dem behandelnden Arzt ein optisches öder akustisches Signal angegeben werden, welches ihn während der Durchführung der Behandlung unterstützt und die weitere Durchführung der Ablation, beispielsweise anhand von EKG-Daten, nicht behindert.

Wird bei sehr hoher Energieabgabe die Verdampfung oder Vaporisierung innerhalb des zu behandelnden Gewebes bewirkt, kommt es in der.Regel zur Ausbildung einer Spannungsspitze, die als Peak 20 in Fig. 1 gemessen wurde. Derartige Vorgänge sind gemäß der Erfindung durch Erfassung des zeitlichen Differentials und/oder des Absolutwertes des Potentialverlaufs oder durch eine Funktion in Abhängigkeit von beiden gemessenen Werten als Steuergröße verwendbar, um die Zufuhr von Hochfrequenzenergie zu unterbrechen oder zumindest lokal dem Vorgang zugeordnet zu vermindern.

Es liegt ferner im Rahmen der Erfindung, die Steuereinrichtung 16 mittels eines externen Rechners oder eines Personal-Computers 19 zu realisieren.

Die Erfindung ist ebenfalls nicht auf die Hochfrequenz-Katheterablation beschränkt, sondern kann bei den meisten anderen Katheterablationsverfahren mit Erfolg zur Überwachung des jeweiligen Behandlungserfolgs verwendet werden.

Zusammenfassend können mit der erfindungsgemäßen Vorrichtung insbesondere folgende Verfahren durchgeführt werden.

Verfahren zur Erfassung der Wechselwirkung und/oder des Kontaktes eines in einem Gefäß eines Patienten, insbesondere in der Blutbahn eines Patienten, angeordneten Katheters zu/mit dem Gewebe des Patienten, wobei die beim Inkontakttreten und/oder Inkontaktstehen einer Elektrode des Katheters mit dem Gewebe des Patienten auftretenden Spannungssignalen erfasst werden.

Verfahren, wie vorstehend erläutert, dadurch gekennzeichnet, daß das Erfassen der Spannungssignale deren Messung mit einer hochohmigen Meßeinrichtung, die vorzugsweise einen Eingangswiderstand von wenigstens 500 kOhm aufweist, umfaßt.

Verfahren zur HF-Katheterablation, bei welcher Gewebeabschnitte eines zu behandelnden Patienten durch Einstrahlung von Hochfrequenz abladiert werden, ferner umfassend ein Verfahren, wie vorstehend erläutert zur Erfassung des Kontaktes zwischen dem Katheter und dem Patientengewebe und/oder der Temperatur des Patientengewebes.

Verfahren zur HF-Katheterablation, wie vorstehend erläutert, dadurch gekennzeichnet, daß der zur Ablation verwendete Katheter über mehrere, separat ansteuerbare Ablationselektroden verfügt, an welchen die Spannungssignale auftreten, oder weitere separat angeschlossene Elektroden umfaßt, die jeweils Ablationselektroden zugeordnet sind, wobei an den separaten Ablationselektroden oder an den zugeordneten Elektroden die Spannungssignale erfaßt werden.

Verfahren zur HF-Katheterablation, wie vorstehend erläutert, dadurch gekennzeichnet, daß die Spannungssignale während der Durchführung der HF-Ablation erfaßt werden, wobei die Hochfrequenzsignale zur Ablation entweder kontinuierlich oder gepülst angelegt werden.

Verfahren zur HF-Katheterablation, wie vorstehend erläutert, dadurch gekennzeichnet, daß die Spannungssignale während der Abgabe der Hochfrequenzleistung erfaßt werden und bei Unterschreiten oder Überschreiten eines Grenzwertes die Abschaltung oder zumindest die Verminderung der Abgabe der Hochfrequenzleistung bewirken.

Verfahren zur HF-Katheterablation, wie vorstehend erläutert, dadurch gekennzeichnet, daß die von dem Katheter abgegebene Hochfrequenzleistung zeitlich integriert wird, solange die Spannungssignale oberhalb eines vorgegebenen Grenzwertes liegen und die integrierte Leistung als bisher in das Gewebe abgegebene Ablationsenergie berechnet und dargestellt wird.

Verfahren zur HF-Katheterablation, wie vorstehend erläutert, dadurch gekennzeichnet, daß bei Erreichen eines vorgegebenen Ablationsenergiewertes die Abgabe von Hochfrequenzleistung an die zugehörige Katheterelektrode abgeschaltet oder zumindest vermindert wird.

Verfahren zur HF-Katheterablation, wie vorstehend erläutert, dadurch gekennzeichnet, daß der Verlauf der Spannungssignale sowie der abgegebenen und/oder der zeitlich integrierten Ablationsleistung den jeweiligen Katheter-Elektroden zugeordnet aufgezeichnet und gespeichert wird.

Verfahren zur HF-Katheterablation, wie vorstehend erläutert, dadurch gekennzeichnet, daß die auftretenden Spannungssignal optisch und/oder akustisch angezeigt werden, wobei die optische Anzeige in Form von Balkendiagrammen, Echtzeitdarstellung der Spannungssignale in einem zweidimensionalen Koordinatensystem und/oder in einer Anzeige von zeitlich integrierten Leistungsdaten erfolgt.

## Patentansprüche

1. Vorrichtung zur Erfassung der Wechselwirkung und/oder des Kontaktes eines in einem Gefäß eines Patienten bei der Katheterablation, insbesondere in der Blutbahn eines Patienten, angeordneten Katheters zu/mit dem Gewebe des Patienten, umfassend eine Katheterelektrode (a, b) und eine weitere Elektrode (a, b, 4) sowie eine Einrichtung (1) zur Erfassung einer Spannung zwischen der Katheterelektrode (a, b) und der weiteren Elektrode (a, b, 4) derart, dass die Spannung als Maß für den Kontakt der Katheterelektrode (a, b) zum Gewebe und zur Temperaturmessung benutzbar ist,
wobei die weitere Elektrode eine mit dem Körper des zu behandelnden Patienten in Kontakt stehende, indifferente Elektrode (4) oder eine weitere an dem Katheter (3) angebrachte Elektrode (a, b) ist.

2. Vorrichtung nach Anspruch 1, **dadurch gekennzeichnet, daß** die an die Spannungsmeßeinrichtung (1) angeschlossenen Katheterelektroden (a, b) und die indifferente Elektrode (4) aus Platin bestehen oder mit Platin beschichtet sind.

3. Vorrichtung nach einem der Ansprüche von 1 oder 2, **dadurch gekennzeichnet, daß** die Spannungsmeßeinrichtung (1) einen Eingangswiderstand von wenigstens mehr als 100 KOhm und vorzugsweise mehrere MOhm aufweist.

4. Vorrichtung nach Anspruch 3, **dadurch gekennzeichnet, daß** die Spannungsmeßeinrichtung (1) einen Eingangswiderstand von etwa 1 MOhm und eine Eingangskapazität von nicht mehr als 30pF aufweist.

5. Vorrichtung nach einem der Ansprüche von 1 bis 4, **dadurch gekennzeichnet, daß** die Messung der Spannung vermittels der Spanungsmeßeinrichtung (1) während der Abgabe der Hochfrequenz-Ablationsleistung durchführbar ist.

6. Vorrichtung nach einem der Ansprüche von 1 bis 5, **dadurch gekennzeichnet, daß** der Ablationskatheter (3) mehrere aus Platin bestehende oder mit Platin beschichtete Hochfrequenz-Ablationselektroden (a, b) umfaßt, an welchen die Spannungssignale zur Erkennung des Katheter-Gewebekontaktes und/oder der Gewebetemperatur abgegriffen werden.

7. Vorrichtung nach einem der Ansprüche von 1 bis 6, ferner **gekennzeichnet durch** ein Tiefpaßfilter (2) zur Unterdrückung von Störungen der Hochfrequenz-Ablationssignale sowie von Brummstörungen.

8. Vorrichtung nach einem der Ansprüche von 1 bis 7, ferner **gekennzeichnet durch** eine Einrichtung (15) zur Aufzeichnung und Speicherung der Spannungssignale, sowie vorzugsweise auch der abgegebenen Hochfrequenzablationsleistung und Energie.

9. Vorrichtung nach Anspruch 8, **dadurch gekennzeichnet, daß** der Verlauf der Spannungssignale sowie der momentanen abgegebenen und/oder der zeitlich integrierten Ablationsleistung den jeweiligen Katheterelektroden (a, b) zugeordnet aufgezeichnet und gespeichert wird.

10. Vorrichtung nach Anspruch 8 oder 9, ferner **gekennzeichnet durch** eine Einrichtung (16, 19) zur Anzeige und/oder Auswertung der aufgezeichneten Signale, **durch** welche die auftretenden Spannungssignale optisch und/oder akustisch angezeigt werden, wobei die optische Anzeige vorzugsweise in Form von Balkendiagrammen, Echtzeitdarstellung der Spannungssignale in einem zweidimensionalen Koordinatensystem und/oder **durch** die Anzeige von zeitlich integrierten Leistungsdaten erfolgt.

11. Vorrichtung nach einem der Ansprüche von 1 bis 11, ferner **gekennzeichnet durch** eine Einrichtung (15, 16, 19), **durch** welche die Spannungssignale während der Abgabe der Hochfrequenzleistung erfaßt werden und, falls die Spannungssignale einen vorgebbar einstellbaren Grenzwert (U_{g}, 17) unterschreiten, die Abschaltung oder zumindest die Verminderung der Abgabe der Hochfrequenzleistung bewirkt wird.

12. Vorrichtung nach einem der Ansprüche von 1 bis 11, ferner **gekennzeichnet durch** eine Einrichtung (16, 19), **durch** welche die von dem Katheter abgegebene Hochfrequenzleistung zeitlich integriert wird, so lange die Spannungssignale oberhalb eines vorgegebenen Grenzwertes (U_{g}, 18) liegen und **durch** welche die integrierte Leistung als bisher in das Gewebe abgegebene Ablationsenergie oder erreichte Wechselwirkung mit dem Gewebe oder erreichte Ablationstiefe im Gewebe berechnet und dargestellt wird.

13. Vorrichtung nach Anspruch 12, ferner **dadurch gekennzeichnet, daß** bei Erreichen eines vorgegebenen, bisher in das Gewebe abgegebenen Ablationsenergiewertes oder erreichter Wechselwirkung mit dem Gewebe oder erreichter Ablationstiefe im Gewebe die Abgabe von Hochfrequenzleistung an die zugehörige Katheterelektrode abgeschaltet oder zumindest vermindert wird.

## Claims

1. Device for detecting the interaction and / or the contact of a catheter arranged in a receptacle of a patient during catheter ablation, particularly in the bloodstream of a patient, with the tissue of the patient, comprising a catheter electrode (a, b) and a further electrode (a, b, 4) and a device (1) for detecting a voltage between the catheter electrode (a, b) and the further electrode (a, b, 4) with the result that the voltage can be used as a measure for the contact of the catheter electrode (a, b) with the tissue and for temperature measurement,
wherein the further electrode is an indifferent electrode (4) in contact with the body of the patient to be treated or a further electrode (a, b) connected to the catheter (3).

2. Device according to claim 1, **characterised in that** the catheter electrodes (a, b) connected to the voltage measuring device (1) and the indifferent electrode (4) consist of platinum or are coated with platinum.

3. Device according to one of the claims 1 or 2, **characterised in that** the voltage measuring device (1) has an input impedance of at least more than 100 KOhm and preferably several MOhm.

4. Device according to claim 3, **characterised in that** the voltage measuring device (1) has an input impedance of about 1 MOhm and an input capacitance of not more than 30pF.

5. Device according to one of the claims 1 to 4, **characterised in that** the measurement of the voltage can be carried out by means of the voltage measuring device (1) during the output of the high frequency ablation power.

6. Device according to one of the claims 1 to 5, **characterised in that** the ablation catheter (3) comprises a plurality of high-frequency ablation electrodes (a, b) consisting of platinum or coated with platinum, on which the voltage signals are measured for the purpose of recognition of the catheter / tissue contact and / or the tissue temperature.

7. Device according to one of the claims 1 to 6, further **characterised by** a low-pass filter (2) for suppressing interference in the high frequency ablation signals and humming interference.

8. Device according to one of the claims 1 to 7, further **characterised by** a device (15) for recording and storing the voltage signals and preferably also the output high frequency ablation power and energy.

9. Device according to claim 8, **characterised in that** the pattern of the voltage signals and the ablation power output at any one time and / or integrated in time is recorded and stored in assignment to the respective catheter electrodes (a, b).

10. Device according to claim 8 or 9, further **characterised by** a device (16, 19) for indicating and /or evaluating the recorded signals, through which the voltage signals arising are optically and / or acoustically indicated wherein the optical indication is preferably effected in the form of bar charts, real time representation of the voltage signals in a two-dimensional coordinate system and / or through the indication of output data integrated in time.

11. Device according to one of the claims 1 to 11, further **characterised by** a device (15, 16, 19), through which the voltage signals are detected during the output of the high frequency power and, if the voltage signals fall below a pre-specifiable threshold (Ug, 17), the output of the high frequency power is switched off or at least reduced.

12. Device according to one of the claims 1 to 11, further **characterised by** a device (16, 19), through which the high frequency power output by the catheter is integrated in time so long as the voltage signals lie above a pre-specified threshold (Ug, 18) and through which the integrated power is calculated and represented as ablation energy output into the tissue so far or the interaction achieved with the tissue or the ablation depth reached in the tissue.

13. Device according to claim 12, further **characterised in that** when a pre-specified ablation energy value output into the tissue so far is reached or an interaction with the tissue is achieved or an ablation depth in the tissue is reached, the output of high frequency power to the associated catheter electrode is switched off or at least reduced.

## Revendications

1. Dispositif de détection de l'interaction et/ou du contact d'un cathéter, placé dans un vaisseau d'un patient lors de l'ablation par cathéter, notamment dans le circuit artériel d'un patient, avec le tissu du patient, comprenant une électrode de cathéter (a, b) et une autre électrode (a, b, 4) ainsi qu'un dispositif (1) pour la détection d'une tension entre l'électrode de cathéter (a, b) et l'autre électrode (a, b, 4) de telle sorte que la tension est utilisable comme mesure du contact de l'électrode de cathéter (a, b) avec le tissu et pour la mesure de température,
l'autre électrode étant une électrode neutre (4) en contact avec le corps du patient à traiter ou une autre électrode (a, b) disposée sur le cathéter (3).

2. Dispositif selon la revendication 1, **caractérisé en ce que** les électrodes de cathéter (a, b) raccordées au dispositif de mesure de tension (1) et l'électrode neutre (4) sont en platine ou sont recouvertes de platine.

3. Dispositif selon l'une des revendications 1 ou 2, **caractérisé en ce que** le dispositif de mesure de tension (1) comporte une résistance d'entrée d'au moins 100 kΩ et de préférence de plusieurs MΩ.

4. Dispositif selon la revendication 3, **caractérisé en ce que** le dispositif de mesure de tension (1) comporte une résistance d'entrée de 1 MΩ environ et une capacité d'entrée d'au plus 30 pF.

5. Dispositif selon l'une des revendications 1 à 4, **caractérisé en ce que** la mesure de la tension peut être effectuée au moyen du dispositif de mesure de tension (1) pendant la fourniture de la puissance d'ablation à haute fréquence.

6. Dispositif selon l'une des revendications 1 à 5, **caractérisé en ce que** le cathéter d'ablation (3) comprend plusieurs électrodes d'ablation à haute fréquence (a, b) qui sont en platine ou recouvertes de platine et sur lesquelles les signaux de tension sont prélevés pour la détection du contact entre cathéter et tissu et/ou de la température de tissu.

7. Dispositif selon l'une des revendications 1 à 6, **caractérisé en outre par** un filtre passe-bas (2) pour la suppression de perturbations dans les signaux d'ablation à haute fréquence ainsi que de perturbations de ronflement.

8. Dispositif selon l'une des revendications 1 à 7, **caractérisé en outre par** un dispositif (15) pour l'enregistrement et la mémorisation des signaux de tension, ainsi que de préférence de la puissance et de l'énergie d'ablation à haute fréquence fournies.

9. Dispositif selon la revendication 8, **caractérisé en ce que** l'allure des signaux de tension ainsi que de la puissance d'ablation momentanément fournie et/ou de la puissance d'ablation intégrée sur le temps est enregistrée et mémorisée avec une référence aux électrodes de cathéter respectives (a, b).

10. Dispositif selon la revendication 8 ou 9, **caractérisé en outre par** un dispositif (16, 19) qui est destiné à l'indication et/ou à l'évaluation des signaux enregistrés et par lequel les signaux de tension apparaissant sont indiqués de manière optique et/ou acoustique, l'indication optique s'effectuant de préférence sous la forme de diagrammes en bâtons, représentation en temps réel des signaux de tension dans un système de coordonnées à deux dimensions et/ou par l'affichage de données de puissance intégrées sur le temps.

11. Dispositif selon l'une des revendications 1 à 11, **caractérisé en outre par** un dispositif (15, 16, 19) par lequel les signaux de tension sont détectés pendant la fourniture de la puissance à haute fréquence et qui, si les signaux de tension deviennent inférieurs à une valeur limite (U_{g}, 17) réglable d'une manière pouvant être prescrite, provoque la coupure ou au moins la réduction de la fourniture de la puissance à haute fréquence.

12. Dispositif selon l'une des revendications 1 à 11, **caractérisé en outre par** un dispositif (16, 19) par lequel la puissance à haute fréquence fournie par le cathéter est intégrée sur le temps tant que les signaux de puissance se trouvent au-dessus d'une valeur limite (Ug, 18) prescrite et par lequel la puissance intégrée est calculée et représentée comme énergie d'ablation fournie jusqu'à présent dans le tissu ou interaction obtenue avec le tissu ou profondeur d'ablation obtenue dans le tissu.

13. Dispositif selon la revendication 12, **caractérisé en outre en ce que**, lorsqu'une valeur prescrite d'énergie d'ablation fournie jusqu'à présent dans le tissu ou d'interaction obtenue avec le tissu ou de profondeur d'ablation obtenue dans le tissu est atteinte, la fourniture de puissance à haute fréquence à l'électrode de cathéter associée est coupée ou au moins réduite.
